# EUROPEAN PATENT APPLICATION

(11) **EP 2 511 379 A1**
(43) Date of publication of application: **17.10.2012**
(21) Application number: 12163825.8
(22) Date of filing: 11.04.2012
(51) Int. Cl.: C12Q 1/22, G01N 31/22

(54) **Time and temperature monitoring device**

(30) Priority: 11.04.2011 ES 201130572
(71) Applicant: Universidad Pública De Navarra, 31006 Pamplona, Navarra (ES); Centro Nacional De Tecnologia Y Seguridad Alimentaria. Laboratorio Dei Ebro, 31570 San Adrian, Navarra (ES)
(72) Inventor: Ruete Ibarrola, Leyre, 31006 Pamplona, Navarra (ES); Arregui San Martín, Francisco Javier, 31006 Pamplona, Navarra (ES); Matías Maestro, Ignacio Raúl, 31006 Pamplona, Navarra (ES); Aldaz Donamaría, Paula, 31006 Pamplona, Navarra (ES); Socorro Leranoz, Abián B., 31006 Pamplona, Navarra (ES); Virto Resano, Raquel, 31570 San Adrián, Navarra (ES); Saiz Abajo, María José, 31570 San Adrián, Navarra (ES)
(74) Representative: Elzaburu Marquez, Alberto

(57) **Abstract**

Time and temperature monitoring device. The device makes it possible to monitor the time and temperature conditions in which the product to which it is adjoined has been kept, by means of a gradual change in the same, so that it may be used to control the cold chain of foods and medicines. It is based on the growth of microorganisms above a threshold temperature, which gives rise to an acidification that causes a visible change in a substance that is present in the medium, such as a change of colour. The microorganisms and the colourimetric indicator are held within a porous matrix, preferably cellulose, with a basic weight equal to or greater than 100 g/m², placed between two sealed plastic sheets, one of which is transparent. This porous matrix has optimum characteristics to permit, without the presence of a texturising agent, optimum and homogeneous visualisation of the change which has arisen within the device.

## Description

### TECHNICAL FIELD

The present invention refers to a device capable of monitoring the cumulative time-temperature history of a product. More specifically, the invention refers to a time temperature device based on the growth of microorganisms and the structural elements of the same, which make possible the homogeneous growth of the microorganisms. This leads to a change in the medium which is observed thanks to the presence of an indicator, wherein the change in the medium leads to a modification of some characteristic, easily perceived by the human eye. This kind of devices can be used to indicate the exposure to excessive temperatures for a certain time (or a long exposure above some threshold temperature) of agri-food products, pharmaceutical and biomedical products.

### BACKGROUND OF THE INVENTION

Food spoilage inevitably occurs as time goes by. Spoilage rate depends on the features and physical structure of foods themselves, on the present microorganisms and on the environmental conditions of conservation. Other factors can also change the spoilage rate as for example: food acidity, the respiratory rhythm of the microorganisms, content of nutrients, natural resistance against microorganisms and the biological structure. The spoilage process of food is determined by the above mentioned features as well as the storage conditions.

A qualitative analysis may be enough to detect changes on food properties due to this alteration process. However, performing such analysis may not be always available. Transportation and storage conditions are closely linked to the food spoilage rate and, due to this, there is a growing trend of controlling the internal conditions of the food package in order to achieve the optimal storage conditions. Modified Atmosphere Packages (MAP), which employ nitrogen gas or a mixture of nitrogen and carbon dioxide to reduce oxygen levels below the 1%, is an example of these technologies. In these packages, it is of pivotal importance that the package remains unaltered to avoid carbon dioxide leakages and oxygen entrance. Due to this, qualitative analysis of the food, which requires direct contact with the product, cannot be conducted.

One of the factors that have allowed to prolong the lifetime of food is the introduction of the cold chain. The cold chain is defined as the necessary sequence of elements and activities to guarantee the quality of a foodstuff from its natural or precooked state to consumption. This definition also applies to the sanitary products where quality is ensured from production to consumption. Some of the pharmaceutical or medical related products needing the use of the cold chain are: biological preparations, clinical analysis, organs for transplantation, insulin, eye drops, blood products for transfusion, vaccines... As a general affirmation, the 20% of the most sold pharmaceutical products are sensitive to temperature. Almost the 100% of vaccines and the 68% of products commercialized by biotechnological companies need storage and transportation in the range from 2 to 8 °C. Well known examples of a sanitary product needing a cold chain for preservation are vaccines. Vaccines, as others biological substances, suffer an accelerated process of degradation and deterioration when exposed to heat, destroying the active principle or the immunizing antigen. The cold chain must be respected in the whole process from fabrication to administration. Vaccines are thermolabile biological products that must be conserved between +2°C and +8°C. Higher temperatures can lead to a loss of activity, which inactivation may occur in some cases without changes on the vaccine appearance. Certain vaccines are more sensitive to heat than others. Once the vaccine has lost its effectiveness, placing it in a fridge or a freezer does not allow to recover it. The vaccine activity loss is variable and depends on the reached temperature thresholds as well as the period of time that the vaccine has been exposed to these temperatures. In some cases, vaccines change their appearance or modify their physicochemical features, for this reason it is important that the personnel in charge of the immunization process is properly educated, knows well the features of the vaccines that they administer as well as the storage and conservation rules. These premises cannot always being assured so Time Temperature Indicators (TTI) may serve as an indicator of the cold chain rupture.

The cold chain is used for more than a half of the consumed food in several countries. In addition, it also exists a growing demand for expanding the lifespan of food which implies that new risks may emerge.

As previously commented, food conservation by cold is one of the most spread and accepted methods for food conservation. The conservation techniques by cold can be divided in two types:
■ Freezing: Freezing implies temperatures below 0°C and allows long-term conservation of food.
■ Refrigeration. The term refrigeration relates to temperatures above 0°C and gets food conservation periods which ranges from days to weeks at most.

One of the main reasons of the positive effect of applying cold as a conservation technique is that cold inhibits partially or totally the growing of altering agents. It is so because the growth rate of microorganisms is highly influenced by temperature: Growth rate decreases as the temperature is reduced. There exists a minimum temperature value, different for each one, and below this value microorganism cannot growth. In general, the growth rate increases with temperature, fundamentally due to an overall elevation of the enzymatic reaction rate in microorganisms, up to an optimal temperature, specific for each class of microorganism, when the growth rate reaches a maximum. An abrupt decay on the growth rate appears as the temperature rises above this optimal temperature until reaching a maximum temperature which leads to the cellular death. In this way, if the temperature decreases then growth rate also decreases and the metabolism of the microorganisms is reduced to the extent that cells can stop growing.

Microorganisms are classified taking into consideration their minimum, optimal and maximum growth temperature.

| Type of Microorganism | Minimum T^{a} | Optimal T^{a} | Maximun T^{a} |
|---|---|---|---|
| Psychrophile | (-5) - (+5) | 12 - 15 | 15 - 20 |
| Psychrotroph | (-5) - (+5) | 25 - 30 | 30 - 37 |
| Mesophile | 5 - 15 | 30 - 45 | 35 - 47 |
| Thermophile | 40 - 45 | 55 - 75 | 60 - 90 |

This is not a strict classification as the temperature range (minimum, optimal and maximum) of mesophilic microorganisms is frequently expanded in other classifications, where psychrotrophs are considered as a special class of mesophiles, able to grow at low temperatures. There also exist bibliographic references where some special psychrophilic bacteria, named cryophiles, are distinguished, which are able to multiply below 0°C.

According to this classification, it can be easily deduced that, at ambient temperature, mesophilic microorganisms are the main agents responsible for spoilage of food and other products sensitive to their action. However, when food and other perishable products are refrigerated, or even frozen at slightly below zero temperatures, psycrophilic and psycrotrophic microorganisms, mainly, can maintain their activity in said products, although their growth rate is cut down.

For the particular case of food, refrigeration or freezing can stop or slow down the bacterial activity, but they do not eliminate it so, when the product is heated, the bacterial activity is restored. If temperature is cut down again, bacterial activity will be inhibited again but, compared to the initial situation prior to the heating of the food, the total amount of bacteria will be increased. This process is repeated each time the storage temperature is raised. It must be taken into consideration that, at the time that the product is consumed, as the number of bacteria present on it is higher, the possibility of food spoilage and toxins presence is also higher, considerably increasing the probability of an alimentary intoxication.

The previously exposed reasons have led to a growing interest in the development of dynamic systems that allow consumers to know the state of conservation of food in a safe and reliable manner. Smart tags are one of the possible solutions for this problem, because they are able to modify their state as a function of the food conservation state, indicating whether it is able for consumption or not.

Among smart tags, the Time Temperature Indicators (TTI) are the most widespread. The TTI devices are used to control the preservation of the cold chain of food products whose processing has to assure that a critical threshold temperature is not reached during the period ranging from food fabrication to consumer. At the same time TTI must trace that in the period previously mentioned the food products are suitable for consumption. Depending on the change that takes place in the tag to highlight the product unsuitability for consumption, there exist different types of TTI devices:

### Thermochromic inks-based devices:

TTI devices based on thermochromic inks are usually coloured or transparent pills, containing an area that will suffer a change of colour if the cold chain has been disrupted.

The operating temperature range may be very wide, as there exist inks which vary their colour from -25 °C to 66 °C. **The activation temperature** is known as the specific temperature at which the change of the colour takes place. In order to avoid undesired activations of these devices, they must be stored at lower temperatures than their activation temperature.

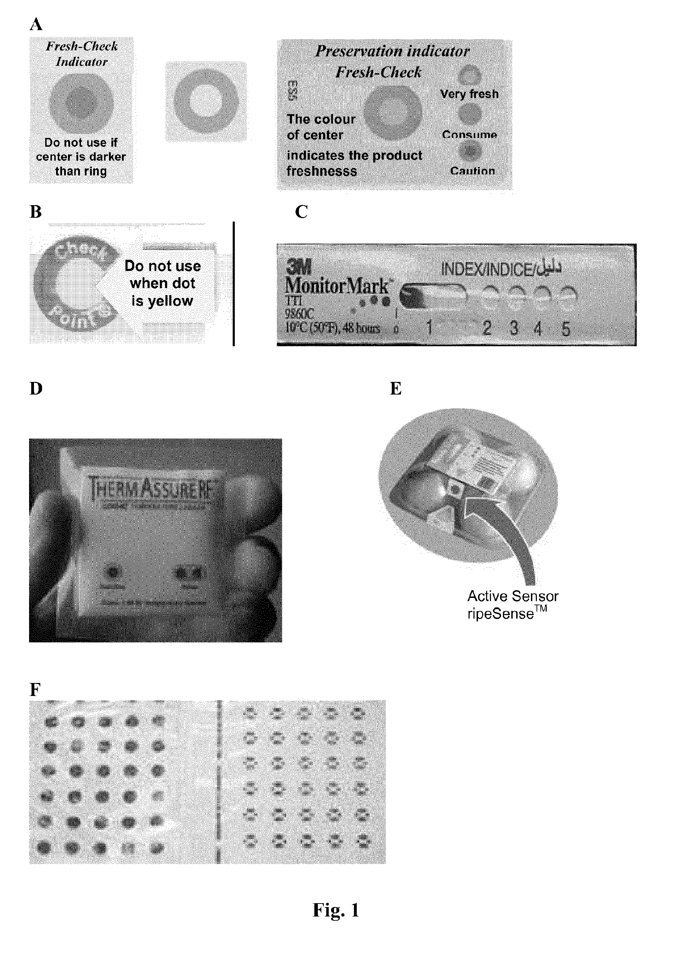

An example of this technology is the Fresh-Check® TTI, which is commercialised by **Temptime** (formerly known as **Lifelines Technologies),** currently available in yellow and red, as it is shown in Fig.1A. A darkening of the central area with regard to the ring implies that the product is not suitable for consumption.

Other examples of thermochromic inks-based TTI devices commercially available are:
■ ColdMark y WarmMark from Evidencia.
■ CombatFeeding tag
■ Thermostrip and ClearingPoint from Hallcrest
■ Thermax developed by TMC

High price is the main drawback of such devices. Any of the above mentioned examples has a price ranging from $1 to $3.58. Furthermore, these tags must be stored at lower temperatures than their activation temperature. These devices exhibit another disadvantage: the performance may be altered by light exposition, so they must be covered by radiation shielding plastic films. However, no film able to shield efficiently the whole wavelength range affecting the thermochromic inks has been developed up to now.

Some reported patents based in this technology are US20060127543, EP0837011, GB2455699, KR100890863, US2006249949, GB2373338, EP0779353. Other type of thermochromic inks are those based on leuco dyes such as those disclosed for instance in: W02008083925, W02008090045, W02009127529, W02009141237, US4917503

### Chemical or Enzimatic reactions based devices

Chemical and enzymatic reactions are strongly temperature dependent. Besides, the reaction kinetics determines the period after which the reaction can be considered completed. Due to this, certain chemical or enzymatic reactions can be used to monitor a time -temperature combination, as TTI devices do.

In some cases, chemical and enzymatic reactions generate some products that can be employed to quantify the time and temperature conditions of the reaction.

Some market available products which use this technology are:
■ CheckPoint® tags, with their three versions, from Vitsab. An example can be seen in Fig. 1B, where it can be appreciated the similarity between this design and the presented in Fig. 1A, which is based on the change of colour of the central area surrounded by an external ring, although the performance principle differ in both cases.

Reagents must be kept apart or inactive until the activation instant, which is the main problem exhibited by these tags. Some of these tags must be stored at refrigeration temperatures. In other cases, the chemical reagents participating in the reaction must be kept physically separated using a barrier, which is eliminated by pressure and rupture or by a melting process which happens at the activation instant of the device.

Numerous patent documents based on chemical and enzymatic reactions to measure time as a function of temperature exist. Some of them are cited below: US4834017, FR2712084, US3942467, US4298348, US4812053, EP0497459, W00127608, W02005075978, W02006015961, W02006015962, W02006091464, W02006091465, W02006091465, W02006091466, W02007035365, W02005075978,

### Diffusion based devices

There exist TTI devices whose performance is based on the diffusion of a compound through a porous material. The product has a specific melting temperature which coincides with the critical temperature of the product. The measurable response is the progression of the melted product from the origin. The useful range of temperatures and the response time of the TTI device depends on the concentration and type of the melting product.

Several devices employing this technology are commercially available such as:
■ TTI MonitorMark™ and FreshnessCheck™ from 3M

A photograph of the first of these examples can be seen on Fig. 1C.

The principal drawback of these devices is their elevated price, which can exceed $2 per unity.

Furthermore, a preconditioning process previous to their use is required in order to assure a correct performance, during which preconditioning process the tag must be kept below a certain temperature which is indicated in their reverse. A security strip must be removed in to activate the device. The system accuracy is 1 °C for temperature and an 8% for time.

There are numerous examples of patents that make use of this technology as for instance EP0484578, EP0930487, W08301834, W02007116071, EP0606033, EP0461044, EP0930489, W09956098

### RFID tags

RFDI tags (Radio Frequency Identification Device) seems to be the more sophisticated solutions. RFIDs are prepared to receive a radiofrequency signal and immediately forward a different radiofrequency signal containing the pertinent information as a response.

There are many commercially available RFID tags. Nevertheless, only some of them can monitor temperature. Some examples of such tags are:
■ Therm Assure RF from the company Evidencia, shown in Fig. 1D
■ Variosense from the company KWS

RFID tags are not widespread nowadays, but shortly products with added value will be fitted out with them, finally arriving at food products. An increase in production volume should lead to a decrease in price.

Up to now some patent document related to TTI devices using RFID technology have been published, for example: W02008053117.

### Aroma detector devices

The New Zeland group Jenkins has developed, in collaboration with the HortResearch Institute, a single use smart tag which detects ripeness of fruit: ripeSense™, that is shown in Fig. 1E.

Flavor and odor of fruit are closely linked to its ripeness. Using this evidence, the developed system consists on an aroma sensor that is integrated on a tag which is place on a rigid plastic package.

In addition to ripeSense™, other devices focused on fruits and vegetables ripeness that are based on ethylene detection have been disclosed in patent documents: US2006127543.

### Biosensors

Biosensors base their operation on the detection of substances related to food spoilage. These substances can be volatile, as in the case of fish spoilage when nitrogen volatile bases are released, or suspended substances in a liquid. There exist several biosensors based on the detection of different microorganisms species which are linked to the decomposition process of food, as in alimentary intoxications. Most of biosensors are not yet commercially available. However, there exist two kind of biosensors that can be currently found at market:
■ ToxinGuard (see Fig. 1F): Developed by the enterprise ToxinAlert
■ FoodSentinelSystem: That is commercialized by Sira Technologies

### Electronic sensors

Some electronic sensors capable of monitoring the temperature record, may be the base of TTI devices as are described in the patent documents W02009156285, W02008083926, W02004097357, US2007076779, EP0060947, W02007074247, W00026111

### Microorganisms based TTI devices

As it has been mentioned before, food suffers a degradation process as a function of temperature due to the development of microorganisms which are naturally present at products. In the same way, vaccines lose their properties if they are not stored under ideal conditions. The use of microorganisms to simulate the real spoilage process of products, as these microorganisms can develop in the same temperature conditions and even, similar velocity of those microorganisms that spoil said products, have led to different devices and patents.

These devices share some features as:
● The utilization of microorganisms able to grow in the range of temperatures that the product will be exposed to. These microorganisms can be bacteria or yeasts.
● Specific nutrients for the microorganism present at the device.

A change in the medium due to the microorganism growth is the base of the performance of such devices. In some cases, the change in the medium is enough to carry out the desired response, as for example the growth of a microorganism population. In other cases, the addition of a substance, which does not influence the kinetic growth, is necessary to produce a visual signal due to the change in the medium, easy to detect by the final user of the device.

Some of these changes are:
● pH medium change and as a consequence, colour change of a pH indicator. So if the food product is suitable for consumption, the device exhibits a colour which differs from the exhibited colour when it is unsuitable.
● pH medium change and as a consequence, the precipitation of some medium component, as for example, casein. This protein is denaturalized when a decrease of pH occurs, precipitating and making the medium opaque (see the application patent FR2859786 A, which describes the technical features of a tag which resembles those of the commercially available tag TRACEO®).
● Any process impeding that the tag accomplishes the function for which it was designed.

It is compulsory that the device remains inactive until the product is completely processed and brought into the cold chain. There exist several mechanisms allowing the control of the activation moment. Some of them are described in the following paragraph.
● Microorganisms and nutrients separation. The most common way to achieve this separation is creating a physical barrier which disappears at the activation moment.
● Microorganisms and/or nutrient microencapsulation
● Product freezing
● Changes of state from solid to liquid. Nutrients and/or microorganisms are kept lyophilized until de activation moment, moment when they are mixed with a melting compound as the threshold temperature has been reached and allowing the microorganisms growth.
● Conservation under hostile conditions. Microorganisms are kept under conditions inhibiting their growth. One of the possibilities is maintaining the pH of the medium among levels for which the microorganism growth rate is close to zero. At the activation moment, addition of an additional constituent is needed in order to adjust the pH to a desired level and thus permitting the microorganism growth.

In any case, it is necessary that some of the components of the medium are texturising agents, whose function consists of immobilizing the microorganisms and/or nutrients, making the change of the medium properly homogeneous through the device. Some of the texturising agents described in other patents are: carboxymethyl cellulose, hydroxypropyl cellulose, hydroxyethyl cellulose (HEC), enriched agar or pure agar, among others.

An example of a TTI device based on microorganisms is that described in the patent document US20070275467A1, which refers to a method and an indicator device to determine whether a product is suitable to be used or consumed, taking into account that the product is designed to be stored under certain temperature conditions and for a period of time shorter than a given value, so that the product degradation does not discourage its consumption.

The device described in the US20070275467A1 document is a transparent label with reduced thickness and surface, adapted to both the use and the product in which will be adjoined. It comprises a transparent envelop, optionally self-adhesive, which permits to observe any changes produced in the indicator contained in the device. This change will indicate that the product is not suitable for consumption. In order to achieve that the indicator work properly, it is preferably suggested the presence of a microorganism which produces acid during its growth and/or metabolic activity, modifying the pH of the medium. The acid production is observed by the presence of, as an example, a compound that changes its colour as a function of pH, or by the precipitation of the existing casein in the medium. The microorganisms must show an acidification activity in temperature ranges compatible with the product temperatures; the acidification kinetics must also be compatible with those temperatures and preservation periods and be in accordance with the degradation kinetics of the perishable product to control. As an option, lactic acid bacteria are suggested, with preference to the psychrotrophic bacteria (defined as those which metabolic activity carries out between 0°C and 45°C) for the control of the perishable products under refrigeration (between 0°C and 4°C). In order to produce the desired effect, they have to be immersed in an adequate culture medium, and in contact with the constituents leading to the acidification. Moreover, it is commented that the device contains one or more texturising agents in order to obtain the desired appearance and to maintain the medium uniformity. It is suggested the use of thickener agents and gelling agents as well, mentioning as searchable characteristic the ability to impede the flow of the solution which contains the agents. The need to add a texturising agent supposes a complication when fabricating the device. However, its presence does not prevent to consider as convenient the separation between the fabrication process and the activation of the indicator. To this purpose, among other methods, it is recommended that microorganisms are isolated from nutrients, i.e. by encapsulating one of these two elements, which requires the subsequently disappearance of the physical barrier between microorganisms and nutrients, so that the reaction can be carried out indicating that the product is no longer suitable to be consumed. This also supposes an added complication when fabricating the indicator device, which makes it difficult to manufacture it easily at the time that the product is packaged.

US20070275467A1 application, however, makes no mention to the presence, as a part of the indicator device, of a porous solid substrate impregnated by the liquid medium. Neither the Fig. 12 legends of US20070275467A1 application, where one of these devices is represented, mention its presence.

Other patent documents refer to transparent film based devices used for culturing microorganisms and to observe their growth. This is the case of the application US2005084948A1, which describes a device designed to detect the presence of microorganisms in food and the environment, but not designed to act as a time-temperature indicator device coupled to a product which conservation status is to be controlled. Actually, it is a microorganism culture device intended to add to the same a solution obtained from the sample in which the presence of microorganisms is to be checked. The mentioned device consists of a porous matrix layer attached to an adhesive sheet, a water-soluble polymeric layer and a transparent film. The sample solution is added on the porous layer of the matrix and then covered with the transparent film. After that it is incubated for a certain time at a proper temperature for culturing microorganisms. When the sample solution is added on the porous matrix layer, the microorganisms in it are dispersed uniformly throughout the porous matrix layer. Meanwhile, the water-soluble polymeric layer in contact with the porous matrix, is dissolved by the water temporally retained in the porous matrix, so that a high viscosity solution is obtained due to the slow dissolution of the polymer in water, which makes than the polymeric layer and the porous matrix behave as a whole body. Thus, microorganisms are pushed to the surface of the porous matrix layer, place where they form colonies. With respect to the nutrients required for the microorganisms growth, they can be added either to the water-soluble polymeric layer or the test sample itself. Once the water-soluble polymer and the nutrients are dissolved in water, then a good environment for microorganisms is obtained and they may begin to grow.

This device requires, therefore, the presence of a water-soluble polymer, which lead to a high viscosity solution, which provokes the microorganisms to be pushed to the surface of the porous matrix film and that prevents its displacement through it.

Regarding the porous matrix, it is said that it has a basis weight from 40 to 100 g/m², preferably between 50 and 90 g/m², more preferably between 55 and 80 g/m². It is considered inappropriate that the matrix has a basis weight less than 40 g/m², since it could not retain the water and added aqueous solutions. Basis weights greater than 100 g/m² are also discouraged, since they would lead to microorganism growth both on the surface of the porous matrix and inside it, varying the growth rate depending on the location of microorganisms, not giving rise to a uniform distribution of them and making it difficult to observe the microorganism growth in the porous matrix.

In the US2005084948A1 application, air permeability intervals for the porous matrix are also mentioned that are considered adequate for the proper functioning of the device and are calculated by the Japanese standard JIS L 1912 method, standard that affirms that the measurement of the permeability is performed at 13.8 kPa. Thus, US2005084948A1 application mentions that permeability must preferably be from 7 to 24 cm / s (which corresponds, according to the measurement conditions, to the range from 5.07 to 17.39 µm / (Pa . s)), more preferably from 8 to 20 cm / s (5.79 to 14.49 µm / (Pa . s)), and even more preferably 10 to 18 cm / s (7.24 to 3.04 µm / (Pa . s)). It is mentioned that, when the air permeability is less than 7 cm / s (5.07 µm / (Pa . s) in this case), the aqueous components do not disperse adequately, due to the porous matrix lack of water permeability, which can cause problems among which a lack of uniform growth of microorganisms is included. When air permeability is higher than 24 cm / s (17.39 µm / (Pa . )), microorganisms cannot form colonies when growing, making it difficult to count them.

Regarding the materials considered suitable for preparing the porous matrix layer, they are cited in US2005084948A1 application: synthetic fibres (such as nylon fibres, polyacrylonitrile, polyvinyl alcohol (PVA), polyester, polyolefin, polyurethane and ethylene-vinyl acetate copolymer); semi-synthesized fibres such as cotton fibres; natural fibres (including wool, silk, cotton fibres, cellulose fibres and pulp fibres), and inorganic fibres such as glass ones.

With respect to nutrients, no restrictions are imposed, except that they are water-soluble and that they do not inhibit the microorganisms growth, showing preference for the use of peptones, yeast extract, meat extract, casamino acids, amino acids and their mixtures, carbohydrates such as glucose or maltose, and organic acids and their salts. It is mentioned that the presence of salts with ability to control pH (or osmotic pressure) is not a problem.

As can be shown, the device in US2005084948A1 application, apart from not corresponding in the design and application conditions to those of a time-temperature device that can be attached to a food or other perishable product to have an indication of its state of conservation, nor can easily be transformed into one such time-temperature devices, since it requires the addition of nutrients and / or microbial culture inoculum just at the moment in which it is desired to activate the device, this means, to allow the microorganisms growth into it, which either does not make the preparation to be simple nor facilitates that it can take place, for example, at the moment in which the perishable product is packed. It is not considered (because it is not the purpose of this device design), what signal may be an indication that the perishable product to be applied is no longer able to be consumed. It is only explained the conditions in which the microorganisms grow so that their account is easy. Its preparation also requires the use of different materials, not only the biocompatible porous matrix, but also the water-soluble polymer, which has to exhibit a certain viscosity characteristics when mixed with water and the porous matrix for the device to work.

It would be interesting to have a device based on the microorganisms growth, which could be used as a time-temperature indicator, preferably suitable for perishable products that may be conserved under refrigeration or freezing conditions, that may be adapted to an adhesive label structure (to facilitate its product coupling), in which the visualization of the signal discouraging the product consumption was optimal and that it had a simple composition, eliminating the need of components such as texturising agents or water-soluble polymers, so that its fabrication can be simplified and that it was possible even its simple preparation simultaneously to the product package, applying it directly on the same as a part of the packaging process or once it is packaged. Furthermore, it would be especially interesting if that device consisted of a basic structure, which was valid for any time-temperature conditions, that may be used to prepare different versions of the device, each one valid for the control of the accomplishment of certain conservation conditions of the products, conditions that would vary depending on the microorganisms and the culture medium composition and the method used to confirm the accomplishment or breach of the conservation conditions desired for the product.

The present invention provides a solution to this problem.

### SUMMARY OF THE INVENTION

As commented above, the present invention faces the problem of replacing the texturising agent which is used by the time temperature devices based on the growth of microorganisms described in previous applications (eg. US20070275467A1) by an alternative medium which enables the growth of microorganisms, an optimum visualization of the colour change and besides, that allows an easier manufacturing process of the devices.

The solution provided by the present invention is based on using, as a constituent of the device, a solid substrate such as a three-dimensional biocompatible porous matrix which enables the proper growth of microorganisms inside. This growth, in turn, will produce a change in the medium, such as a pH change, which will result in a visible and a homogeneous change of the medium such as a colour change, due to the presence, for instance, of a substance whose colour is modified depending on the pH of the medium. The features of this three-dimensional porous solid substrate, which is in the form of a tridimensional porous matrix, enable the colour change to be sufficiently strong and distinguishable. Within the three-dimensional porous matrix, there is a multilayer structure where the aqueous medium diffuses and circulates freely allowing the medium composition to be homogeneous and the aqueous medium to remain in the entire volume of the multilayer structure. Hence, the growth of microorganisms and the colour change will be homogeneously performed in the whole extension of the porous structure.

These features are obtained by using a biocompatible porous material with suitable physical characteristics in terms of basis weight and, preferably, porosity that allow the colour change to be homogenous and noticeable enough to be distinguishable at a glance by the naked human eye. More specifically, the device comprises a biocompatible porous matrix that has a basis weight equal or higher than 100g/m². Preferably, the air permeability is less than 115 µm/(Pa . s), being able to be greater than 17.39 µm/(Pa . s). Also preferably, the biocompatible porous matrix is based on a cellulosic multilayer structure, having a basis weight equal or above 100 g/m², preferably equal to 480 g/m². The term grammage, characteristic of paper, will be repeatedly used throughout the present application as a synonym of basis weight due for the case of the cellulosic multiplayer structure, which may consist of cellulose mono layers, as both terms refer to the value of the weight per unit of area.

The present device is based on a simple structure which simplifies the manufacture process. Moreover, in order to prevent an accidental activation of the device prior to the adhesion to the potentially monitored product, it is not necessary to prepare the whole device in one go, but the main structure of the device could be initially prepared (the two plastic sheets within the porous matrix, one of them preferably with an adhesive film, the porous matrix being located among said sheets). Both sheets could be partially sealed allowing subsequently the addition of the culture medium, the microorganisms and the pH indicator (in a single solution or separately) through a partial opening. Hence, once all the components are added, the two plastic sheets are completely sealed and the device is adhered to the perishable monitoring product.

Thus, the present invention relates to a device for monitoring the time and temperature history of a product comprising:
a) a first sheet of plastic material that is impermeable to water;
b) a solid porous matrix which is permeable to water whose basis weight is equal or greater than 100 g/m², which is placed on the surface of the first sheet of plastic material;
c) a second sheet of plastic material, which is impermeable to water and transparent or translucent over at least one section of its surface, located over the porous solid matrix, in such a way that the solid porous matrix is covered by the second sheet of plastic material and is visible through the transparent or translucent section of said second sheet of plastic material.

In a preferred embodiment of the invention, compatible with any other one, the first sheet of plastic material contains an adhesive film on the surface of the sheet which is not in contact with the porous matrix, what can be named the "outer" face of the sheet. This facilitates the adhesion of the device to the product to be monitored.

Preferably, both plastic films are sealed to each other, either along the entire perimeter of the device or leaving an unsealed portion which can be sealed afterwards. It is also a possible embodiment that wherein the two plastic sheets are sealed to each other by an adhesive material which allows its subsequent separation.

The solid porous matrix must be made of a biocompatible material which may comprise: cotton fibre, cellulose, wool, silk, keratin, silicon dioxide, glass fibre, polyamide, nylon, polyacrylonitrile, polyvinyl alcohol (PVA), polyester, polyolefin, polyurethane, ethylene-vinyl acetate copolymer, rayon, an spongy material of plant, animal, mineral or synthetic origin, or a mixture of two or more of the materials mention above.

Preferably, the solid porous matrix is a cellulose multilayer structure having a basis weight equal or greater than 100 g/m². It is very suitable for the performance of the device, when the cellulosic structure has a basis weight in the range between 100 and 480 g/m². The results are particularly good in some ranges, such as the range between 150 and 250 g/m², and in particular the value of 480 g/m² whose proper results are shown. These characteristics can be achieved for instance, with porous cellulosic matrices comprising at least 5 cellulosic layers of 24 g/m² each. The porous cellulosic matrices comprising from 5 to 20 cellulosic layers of 24 g/m² each are suitable for the device of the invention, as well as matrices with a higher amount of layers, as for instance, those with values close to 20, such as 21 layers.

It has been observed that particularly good results are obtained when the air permeability of the solid porous matrix is less than 115 µm/(Pa . s), which is a value clearly greater than 17.40 µm/(Pa . s). Proper results are also obtained in case of solid porous matrix of an average porosity greater than 1.18 seconds. Those devices whose basic structures exhibit porous matrices fulfilling any of these conditions are preferred embodiments of the invention. Thus, it is particularly preferred that the air permeability of the porous matrix is below 115 µm/(Pa . s) whatever the material the porous matrix is made of, and/or that the average porosity of the porous matrix to be greater than 1.18 seconds. Within these preferred values of permeability to air, it is also preferred the permeability of the air to be greater than 17.40 µm/(Pa . s).

In a preferred embodiment of the invention, the two plastic sheets are sealed to each other along the whole perimeter of the device. In a particular case of this embodiment, the device further comprises:
d) microorganisms distributed within the porous matrix;
e) a culture medium for said microorganisms, lodged within the solid porous matrix
f) an indicator compound which undergoes a change that is perceptible to the human eye depending on a change in the medium caused by the growth of the microorganisms.

This embodiment of the device, with the two plastic sheets sealed to each other along the whole perimeter, which has the inner part of the label isolated from outside, will be the last version ready to be attached to the product aimed to be monitored. Hence, the time-temperature history of the product will be able to be monitored, such as the cold chain breaking of products to be preserved at refrigeration or freezer temperature.

Preferably the indicator is a substance that changes colour depending on the pH of the medium (such as chlorophenol red) due to the different colour in case of acidic and basic forms of the compound. In addition, one of these forms could be colourless. Thus, the microorganisms distributed by the porous matrix must produce any acid or metabolite during their growth, development phase or metabolic activity that increases the acidity of the medium, for instance, as a result of the fermentation of sugar from the culture medium. This include, for instance, mesophilic, psychrophilic or psychrotrophic bacteria (or even cryophilic), psychrotrophic bacteria being particularly preferred for the products to be conserved between 0°C and 5°C. It is specially preferred that the microorganisms are lactic acid bacteria, which may belong to a single species or may be combinations of several species. Among lactic acid bacteria, *Lactobacillus plantarum* or *Lactobacillus delbrueckii* are particularly preferred.

The culture medium should contain at least the nutrients required by the microorganisms elected, which can be summed up as: carbohydrates (eg. glucose); a nitrogen source (eg. peptone) and, preferably, inorganic salts and/or vitamins or trace elements. It is preferred that the culture medium lacks of substances with buffering capacity, that prevent the pH change in the culture medium.

The medium where the bacteria grow should also contain the indicator substance that undergoes a change detectable by the human eye, such as a colour change depending on pH, which indicates that the product is not in condition for being consumed. This can be achieved by making use of compounds that have different colours in their acidic and basic forms. In addition, one of these forms may be colourless. The indicator must be added to the final device either as part of the liquid culture medium or as an independent solution. The features of the porous solid substrate will favour its homogeneous distribution throughout the device.

Once the device is activated, that is, when it is exposed to a temperature at which the microorganisms present within the device grow, the acidification of the medium will start and as a consequence, the indicator will be modified upon arriving to a certain acidification value. The characteristics of the culture medium, the microorganisms (growth temperature, acid production rate, concentration of microorganisms, mixture of several species thereof) can be adjusted in order to relate the indicator modification to the change of the product to be monitored: the appearance of a substance that it was previously absent in the product (such as a toxic substance which makes no longer advisable to consume the product), a deterioration level that makes the product not advisable for consumption, a condition that advises consuming the product immediately...or even simply to indicate that there has been a break in the intended conditions of product storage for a certain period of time. The change of the indicator compound, such as a colour change, will show that there has been a break in the appropriate storage conditions of the product which the device is attached to, for a period of time long enough to give rise to the condition or to the change in product to be monitored. Hence, the device can be used to monitor whether the minimal conditions required for the preservation of a product that must been kept below a predetermined temperature have been met or not and also to monitor if the minimal preservation conditions have been exceeded for a certain period of time that might have led to a certain degradation level of the product.

Therefore, another aspect of the invention relates to the use of the device of the invention for controlling the time-temperature history of a product which must be preserved under certain temperature conditions, wherein the change in the indicator compound detectable by the human eye indicates that there has been a change in the product conditions (such as the appearance of one or more substances absent previously) or that the product has exceeded a certain temperature threshold for a period of time longer than a predetermined value. As mentioned previously, the resulting change of the indicator compound can be an indication that the product is no longer in a consumable condition, or can be an indication that the product must be consumed immediately or simply, depending on the particular device of the indicator device of the invention, it can be and indication that the storage conditions intended for the product have not been fulfilled.

It is preferred that the change to be observed is the colour change of a pH indicator substance that presents a different colour depending on the pH range of the medium, due to the different colours of its acidic and basic forms.

The product can be any perishable product or any product which can undergo a specific change that needs to be controlled, that is, any product with specific requirements to be preserved under a certain temperature and preferably, a product that undergoes any change to be controlled after exceeding a threshold of a certain temperature for a period of time. The product may be, for instance, a product from the agri-food, pharmaceutical or biomedical sector, such as a food product or a medicament. It is particularly preferred that the product to be monitored is a product that must be preserved under refrigeration conditions, so that the device will be prepared to handle breaks in the cold chain (that will be, in general, breaks in keeping the product between 0°C and 5°C).

### BRIEF DESCRIPTION OF THE FIGURES

Fig. 1 shows several examples of time-temperature labels:
- Panel A: Fresh-Check® labels, based on thermochromic dyes: the degree of darkness into the central circle (minor, equal or major) with respect to the surrounding ring determines, as the label-registered legends indicate, if it is a completely fresh product, if it is still fresh but it has to be rapidly consumed or if it should not be consumed, respectively.
- Panel B: CheckPoint® label, based on chemical reactions: the change of colour of the central circle to yellow indicates that the product must not be consumed.
- Panel C: MonitorMark™ label, in which what it is controlled is the advance from the source of diffusion of a compound whose melting point coincides with the critical temperature of the product. The route of the compound gives a time indication of the time the product has been subjected above the threshold temperature.
- Panel D: ThermAssureRF® label, capable of monitoring the temperature and sendin a radio signal in case of overcoming a certain value. The button on the lower left corner permits to enable or disable the sensor; on the right there is a light emitting diode (LED) which indicates the status: green, right; red (right circle), activated alarm.
- Panel E: ripeSense™ label, which detects volatile compounds related to the fruit maturity and changes its colour depending on its maturity state.
- Panel F: ToxinGuard® label, capable of detecting the presence of a set of substances related to the degradation of food by using specific biosensors capable to interact with them, leading to a reaction which provokes the appearance of a cross if the food state discourages it consumption.

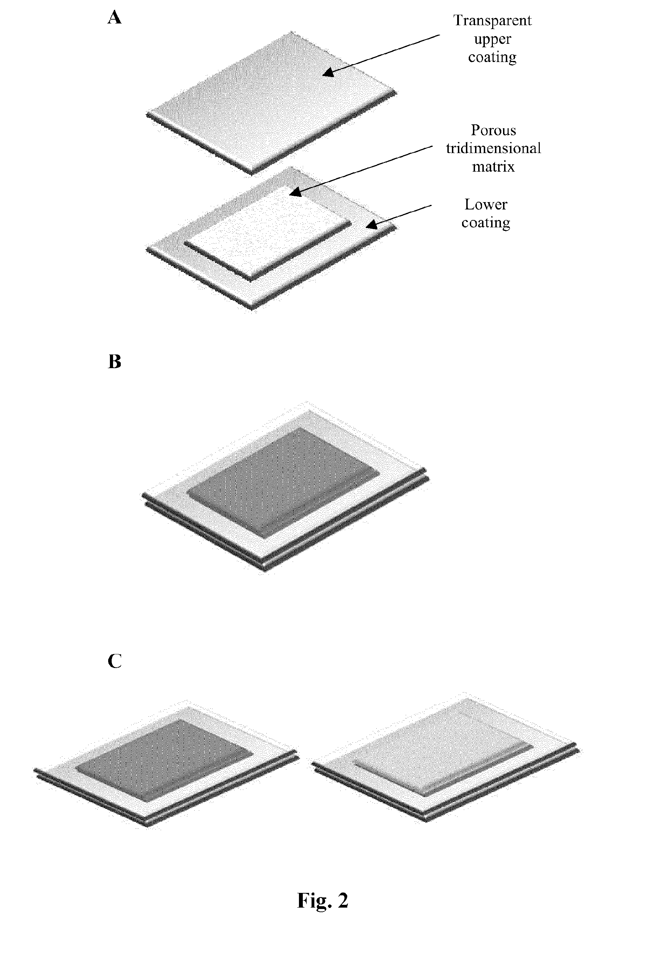

Fig. 2 shows the structure of the device of the invention, designed in order to act as a time-temperature device as a final version, which is the one that is adhered to the product to be controlled.
- Panel A: Basic structure of the device which represents the two plastic films (lower coating and transparent upper coating), that cover the porous tridimensional matrix.
- Panel B: Device structure as a TTI label, once the medium containing microorganisms has been encapsulated into the porous matrix.
- Panel C: Schematic representation of the colour change appreciated in the TTI label of the present invention: on the left, the original colour; on the right, label colour if a break of the cold chain is produced for 6 hours.

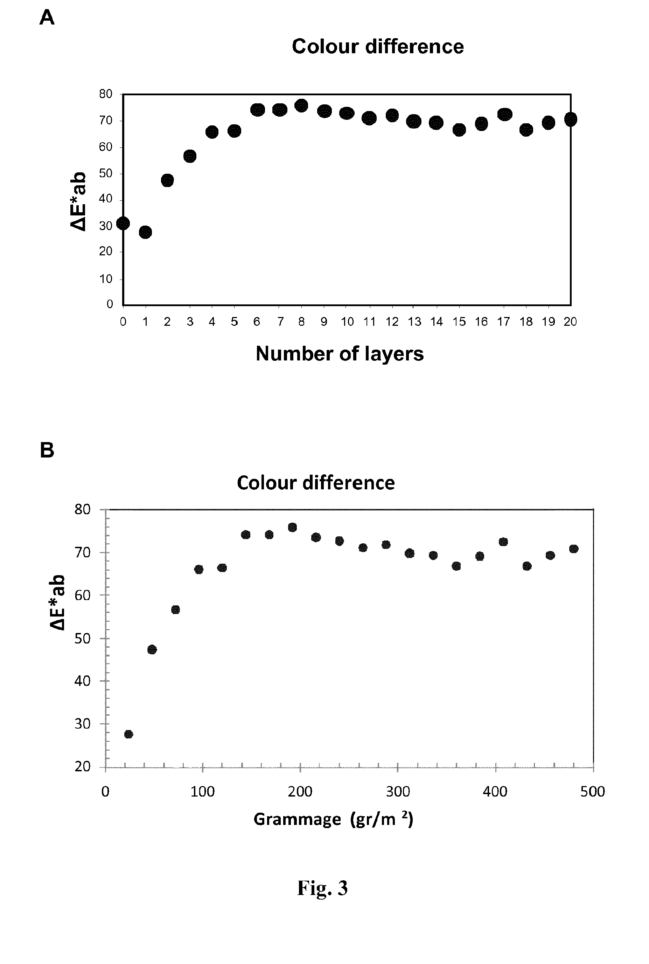

Fig. 3 depicts several schemes in which it is compared the colour difference (ΔE*_{ab}) of the labels of the invention before (initial colour) and after (final colour) the colour change of the dye is produced when having maintained the label at 20°C for more than 6 hours (equivalent to the break of the cold chain), as a function of the structure of the multilayered cellulose matrix:
- Panel A: graphic representing the colour difference as a function of the number of monolayers of 24 g/m² that form the porous matrix.
- Panel B: graphic representing the colour difference as a function of the porous matrix weight (grammage), given in grams per square metre.

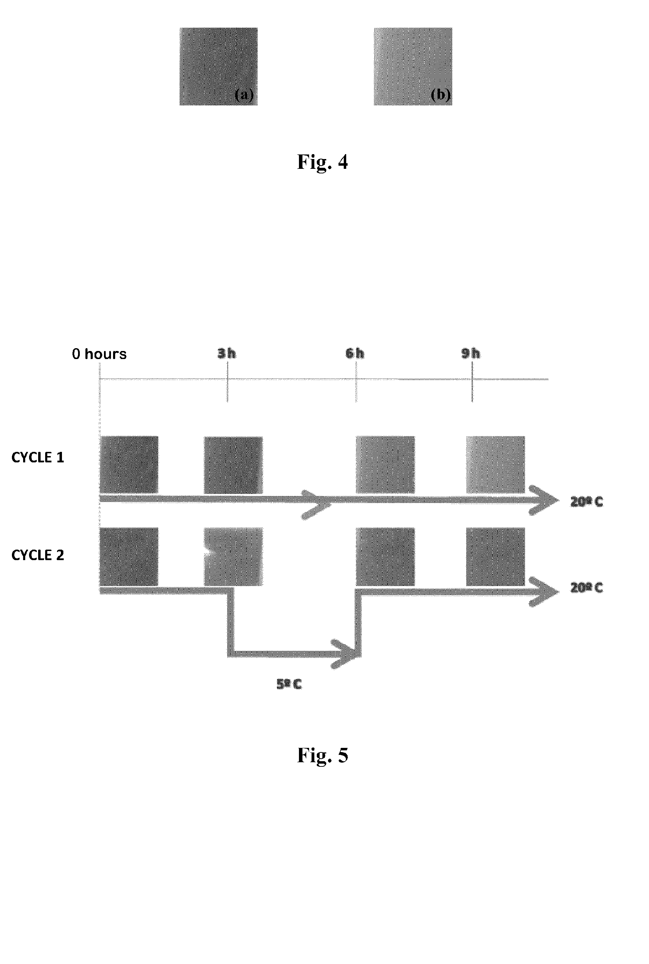

Fig. 4 presents some photographs of the label of the invention, subjected to a continuous cycle of 8 hours to a constant temperature of 20°C, before the turn of colour occurs (photo (a)) and after the break of the cold chain (photo (b)). Part (a), dark gray in the figure, was reddish purple in the original; part (b), brighter grey in the figure, was amber yellow in the original, easily distinguishable from the original colour.

Fig. 5 show some photographs of the devices, obtained every 3 hours when being exposed to the critical temperature of 20°C continuously (cycle 1) or in a discontinuous way, as the line represented at the bottom of the labels indicates: 3 hours (3h) at 20°C, followed by 3 hours at 5°C and afterwards, 3 hours at 20°C (cycle 2). It can be observed that both labels show a reddish brown colour after the 3 first hours of incubation at 20°C. In cycle 1, the label already presents an amber - yellow colour after 6 hours of incubation at 20°C; in cycle 2, it is necessary to take 9 hours of incubation to observe a dark amber colour, although the turn proves that the label presents a memory effect capable to register the temperature excess in a cumulative way.

### DETAILED DESCRIPTION OF THE INVENTION

The invention consist of a time-temperature device, based on the growth of microorganisms, designed to avoid the presence of texturising agents or water-soluble polymers in the culture medium, maintaining optimal conditions for an optical visualization of the change produced at the indicator compound, and in addition, allowing a simpler fabrication of the devices.

Simple manufacturing is one of the main advantages exhibited by the device, making possible to manufacture the device during the packaging process, while this is not strictly necessary. The device shows a fundamental advantage over the prior art devices, which is its low cost, making it applicable to a wide variety of products, increasing their added value.

The proposed solution at the present invention is based on maintaining a "label structure" preferably self-adhesive, similar to the one present at the described indicators in the application patent US20070275467A1, which exhibits two water proof plastic sheets, being at least one of them transparent and the other one having on the exterior face an adhesive layer what eases the positioning on the product to be monitored. Those plastic sheets can be sealed together, so that they serve to contain the culture medium and microorganisms in the final device placed on the perishable product to be monitored and to keep them isolated from the external environment, without preventing the contained microorganisms for being exposed to the same temperature variations experienced by the product on which the label is placed. However, the structure of the devices of the present invention differs from the devices described in the application patent US20070275467A, making not necessary the presence of a texturising agent in the aqueous medium in which microorganisms are expected to grow, in order to obtain the desired appearance and to keep a uniform medium. This is so because the device of the invention has a three-dimensional biocompatible porous matrix, which basis weight is equal or greater than 100 g/m², with an air permeability preferably less than 115 µm/(Pa . s), preferably made of a multilayer cellulosic structure, which features allow the diffusion and free circulation of the aqueous medium where microorganisms grow inside the multilayer structure, so that the medium composition is homogeneous, which is desired, without the necessity of adding a texturising agent to obtain an homogeneous appearance. The features of the porous matrix contained in the device of the present invention ease the aqueous medium to remain within the multilayer structure filling the entire volume, so that the growth of microorganism and the change of colour take place homogeneously in the whole extension of the structure. The porous matrix is biocompatible, so thus it does not pose any impediments for microorganisms to develop, and offers appropriate features for basis weight (grammage) and porosity, to have a perception of the change of colour significant enough for being distinguished at a glance by human eye, (which indicates that the product the device is attached to is not suitable for consumption anymore).

The structure proposed in this invention is novel as it has never been used either in the fabrication of TTI devices or in other systems for monitoring microorganisms' growth which do not involve the fabrication of TTI labels. In these devices for monitoring the growth of microorganisms, a water-soluble polymeric layer is needed to fix bacteria colonies onto the surface of the porous matrix in order to study their growth, as it is described in the patent US2005084948A1. More specifically, in the previously cited patent US2005084948A1 , the required water-soluble polymer, polyvinyl alcohol, is dissolved while coming in contact with the aqueous sample and it is homogenized with the porous matrix, gradually creating a viscous solution that avoids the growth of microorganisms inside the porous matrix. Thus microorganisms remain confined to its surface, facilitating the visual quantification of the growing colonies in the mentioned culture medium. That is, as a difference from the cited application patent US2005084948A1, where the main objective is the growth of colonies on the surface of the porous matrix in order to be quantified, in the present patent application microorganisms grow homogeneously scattered on the whole volume of the three-dimensional porous matrix to obtain an homogeneous change of colour throughout the device too. In this way and as a difference from the patent application US2005084948A1 previously described, the three-dimensional porous substrate of the present invention constitutes a structure wherein the aqueous medium spreads and circulates freely, so thus the medium composition is homogenous.

The structure, preferably multilayered, of the device of the present invention has the particularity that its features differ significantly from the teachings and recommendations for the porous matrixes of the described devices of the patent application US2005084948A1. In fact, the values of basis weight and porosity used in the present application are those discouraged in the patent application US2005084948A1 where they are considered as inappropriate for microorganism growth. More specifically, to accomplish its objective of adequate culture medium, the device described in the patent application US2005084948A1 requieres a porous matrix which basis weight ranges from 40 to 100 g/m², preferably between 55 and 80 g/m², as less than 40 g/m² the porous matrix is unable to retain the water and the added samples, inhibiting the formation of quantifiable colonies. On the other hand, for a basis weight greater than 100 g/m²_{'} the high viscosity water-soluble polymer used in said patent application is retained inside the porous matrix so the viscous solution and the porous matrix do not merge as expected, which, according to the patent application US2005084948A1, allows the microorganism growth both on the surface and inside the porous matrix, varying the growth rate as a function of the location of microorganisms, not leading to a uniform distribution of the same and making difficult to appreciate the growth of microorganisms in the porous matrix. Instead of this, for the device concerning the present patent application, the basis weight of the porous matrix (greater than 100 g/m²) is out of the range cited in the patent application US2005084948A1 as the proper for the growth of microorganisms, despite which the microorganism growth and the change of colour are carried out homogeneously in the entire structure of the porous matrix, without needing a water-soluble polymer in the culture medium. This conditions are also respected for matrixes which basis weight differs significantly from the maximum recommended value in the US2005044948A1 document, as for example basis weights close to 500 g/m² (480 g/m²) exhibited by porous matrixes of the devices employed in the conducted assays that are shown later at the Examples of the present application.

Air permeability is other of the characteristic parameters of the porous matrix for which both inventions may, and preferably, differ. In the matrix of the patent application US2005084948A1 the permeability ranges from 7 to 24 cm/s (values which, according to the measurement conditions, correspond to an interval of 5.07 to 17.39 µm/(Pa.s)), and preferably between 10 and 18 cm/s (from 7.24 to 13.04 µm/(Pa.s)), as below 7 cm/s (5.07 µm/(Pa.s)) the sample is not uniformly merge with the water-soluble polymer as a consequence of the lack of permeability of the porous substrate, preventing a uniform microorganism culture. For permeabilities higher than 24 cm/s (17.39 µm/(Pa.s)), to the contrary, the dissolved water soluble polymer is insufficiently fixed to to the highly viscous solution, thus inhibiting the formation of microorganism colonies easy to observe, and therefore preventing an accurate colonies count. However, in the present application, the air permeability of the described substrate is, preferably minor than 115 µm/(Pa.s), which implies a recommended upper limit which is clearly higher than 17.40 µm/(Pa.s) (more exactly 17.39 µm/(Pa.s), the upper limit recommended in the application US2005084948A1, so the new recommended values are also out of the range of values cited in the application US2005084948A1. In spite of this, the microorganisms are homogeneously distributed throughout the matrix and the change of colour in the pH indicator can be clearly appreciated.

Thereby, the devices of the present invention constitute a TTI system based on microorganisms, which devices can be used, as for example, as cold chain break indicator for those products requiring refrigeration or freezing storage temperatures, including food products, as well as pharmaceutical and veterinary products. The mentioned device solves the problems present in the existing technologies which have been described in the "Background of the invention", relative to the necessity of the presence oftexturising agents or water-soluble polymers.

So that, one of the main advantages of this device is its easy manufacturing process, which allows the manufacturing of the device during the packaging process, while this is not strictly necessary. Moreover, the device also presents another key advantage over the previously disclosed devices, which is its low cost, making it applicable to a wide variety of products, increasing the added value of said products.

A more detailed description of the features of the device is shown afterwards.

### Device preparation and structure. Plastic sheets

The innovarion introduced by the present invention is the structure of the device, which is presented under the form of a flat, preferably self-adhesive label, which exhibits the basic structure depicted in Fig. 2A, comprised of:
● A plastic sheet, preferably having an adhesive layer, which eases the adhesion of the device to the product of interest.
● A porous and permeable solid matrix that will absorb and retain the liquid medium (composed of microorganisms and a culture medium). The matrix retains the liquid medium and acts as a physical support in which the fermentation will be carried out.
● A second plastic sheet that will be placed on both the previously mentioned structures, which must be transparent or translucent permitting to see the porous matrix though it.

As it can be seen in Fig.2A and 2B, the dimensions of the two plastic sheets are preferably similar, as they are designed to be sealed together. In contrast, the solid porous matrix has preferably an area smaller than those of the plastic sheets with which it must be covered, to ease the sealing between them. In a possible embodiment of the device of the invention, wherein the device presents a rectangular shape, the length and the width of the solid porous matrix are smaller than the length and width of the plastic sheets. It is perfectly compatible with the invention that the device exhibits other shapes, as for example circular, in which the diameter of the solid porous matrix is smaller than the diameter of the plastic sheets. Anyway, the device must be smaller than the packaging of the product where the device may be placed, and it may be presented on the product as a little label similarly to the devices shown in Fig.1, so that is the reason why the device of the invention is also named "Time-Temperature label" often throughout the present application.

As it has been above mentioned, it is possible to prepare the basic structure previously, in which initially the plastic sheets may be partially sealed, without sealing the whole perimeter, leaving an opening. It is also possibl, to design the device so that the coating plastic sheets have an adhesive on the faces in contact with the porous matrix, but only on the area which is not in direct contact with the porous matrix (corresponding to the portion of area that the sheets are larger than the matrix), which will allow to fix one sheet to another and to keep the matrix isolated from the external environment. If the basic structure is prepared before the complete device (which is the version of the device which contains, additionally to the basic structure elements, the microorganisms, the adequate culture medium and the indicator substance exhibiting a change perceptible by the human eye as a function of the pH), then the adhesive substance must allow the separation of the plastic sheets when the microorganisms, the culture medium and the indicator are to be added. The combination of all the constituent elements of the final device may also be made at once, at a moment that can be previous to the positioning of the device on the perishable product whose time-temperature record is to be monitored, or the combination of all the constituent elements and the attachment of the time-temperature device can be simultaneous to the product packaging, placing the time-temperature device inmmediately on the product package.

As it has been mentioned at the beginning, it is preferable that at least one of the plastic sheets has an adhesive layer on the face that is not in contact with the porous matrix, face which can be considered as the external face. This makes the positioning on the product to be monitored easier and assures that both product and time-temperature device will be together. The presence of the adhesive layer is not compulsory, as the label can be fixed to the product to be monitored using other techniques, such as using an adhesive tape or an additional transparent adhesive plastic sheet, larger than the label, that may be place on it once the label is placed on the product to which it has to be fixed. In those last cases, it is important to place the label on the product so that the porous matrix is visible, so it is not hidden by, for instance, the adhesive tape; in cases only one of the constituent plastic sheets is transparent or translucent, special attention may be paid to position the plastic sheet allowing to visualize the porous matrix (and thus the changes that may occur on it) to the exterior, not being in contact with the product.

The basic structure of the label, composed of the plastic sheets and the porous matrix between them, has commercial and industrial interest by itself, as it is a basic structure which can be used as the base for the preparation of different embodiments of the final indicator device. These different embodiments may differ in the substance whose change serves as an indicator of not keeping the desired storage conditions for the product, as well as for the present microorganisms (species, amount, temperate growth range, growth rate...) and in the features of the culture medium present at the final and most complete version of the device of the invention. The specific features of these three components will determine the time-temperature conditions for which the indicator device will experiment a perceptible change by the human eye, and thus, the target product to which the indicator device is to be fixed, according to the foreseen storage conditions, threshold temperatures and periods of time, requirements that must be met. At any of these possible embodiments, the presence of a texturising agent is not necessary, as the final indicator device had being prepared according to the basic label structure of the present invention, containing a porous matrix with the above mentioned characteristics.

For the preparation of the final version of the device that can be used as an indicator label of a product, the medium containing the nutrients and the indicator together with the microorganisms are poured on the porous matrix, which at the same time has one of the plastic sheets as support. The culture medium, the microorganisms and the acidification indicator can be independently deposited on the porous matrix, or dissolved (suspended in the case of microorganisms) in an aqueous solution, although it is preferable to mix them before pouring them on the porous matrix and to add them together to the porous substrate.

If the basic initial structure of the label was previously manufactured (the two plastic sheets and the porous matrix between them), the medium containing the nutrients, microorganisms and the indicator will be introduced through the aperture determined by the portion of the sheets unsealed, then the label will be sealed. Alternatively, if the adhesive that kept sealed the plastic sheets allows their separation, the plastic sheets can be separated, as for example by lifting one, and then the culture medium, the microorganisms and the indicator substance are poured on the porous matrix. After that, the other plastic sheet is applied on the outer surface and the edges of the plastic sheets are thermally sealed in order to encapsulate the porous matrix containing the culture medium and the microorganisms in a sandwich type structure between the two plastic sheets as it is represented at Figs. 2A and 2B.

Anyway, at the instant when the totality of the elements that are part of the final version of the device are combined, the sealing of the plastic sheets is needed, so that the inner part of the label is isolated from the external environment and from the product, but being exposed to the same time-temperature conditions than the product where it is placed. So, the device of the invention will be attached to the product package, using, in case it exists, the adhesive layer that may be present on the outer face of one of the plastic sheet, that means, the face which is not in contact with the porous matrix or using an additional adhesive medium allowing the device to be fixed to the product, as it has been previously discussed. Thus, the indicator device will allow monitoring, as for example, the disruption of the cold chain of the product, in case it must be stored under refrigeration conditions or even frozen.

Once the device is completely assembled, if it is not immediately placed on the product to be monitored, it must be stored at low temperatures for preventing the development of microorganisms before being placed on the product. Temperature threshold that may be considered as low enough will depend on the microorganisms present at the device, as it is highly recommended that the storage temperature is kept below the minimum temperature that permits the development of such microorganism. So if the microorganisms are able to grow above 0°C, the device may be kept below this temperature. For a better conservation of the microorganisms, it is recommended to store the device below 0 °C not permitting the presence of liquid water in the medium as for example between (-18 °C) and (-80 °C), which correspond to the temperature ranges commonly used in the food industry and in the domestic sector.

In the case of lactic bacteria preferred for the device of the present invention, *Lactobacillus plantarum* and *Lactobacillus delbrueckii,* the label must be stored at or near to refrigeration temperatures (5°C), which coincides with the refrigeration temperature taken as a reference of a correct refrigeration during the cold chain; in this situation, if the added indicator is chlorophenol red, the label keeps its initial reddish purple colour. As the label is exposed to the critical temperature i.e. when a disruption of the cold chain occurs, which coincides with the optimum growth range for microorganisms, the binary fission will take place. Therefore, the bacteria population will grow and the fermentation of the sugars present at the medium will be carried out, acidifying the medium and causing a change of the colour of the indicator of the device.

The plastic sheet that does not contain the adhesive layer must be transparent, at least partially transparent, that allows to appreciate the porous matrix which retains the liquid medium and the indicator substance that experience a change of colour optically noticeable by the human eye. This change must be significant and easily perceptible by the human eye so it can differentiated in an obvious manner, as it can be seen in the representation of the final device, with all its elements, shown in the Fig.2B, in which the portion corresponding to the porous matrix appear in darker grey as a representation of a coloured matrix by the presence of an indicator substance varying its colour as a function of pH.

Both plastic sheets must be made of a waterproof material, as the culture medium retained in the porous matrix where the bacteria grow is an aqueous medium that must be kept sealed together with the bacteria and the pH indicator.

The sheets could be made of a variety of plastic materials compatible with the invention, such as polyethylene terephthalate (PET), polyethylene (PE) and polypropylene (PP).

### - Microorganisms

As mentioned above, it is preferred for the device the use of a microorganisms population able to produce some kind of acid or metabolite which increases the acidity of the medium through fermentation of any sugar within the liquid medium. In particular, it is preferred that these microorganisms are non-pathogenic and non-toxic. In addition, the growth and acidic substances production must occur at a temperature which is compatible with the storage temperature of the product to be monitored.

The specific bacteria used for each product must be chosen considering the temperature at which the product is to be preserved and the time-temperature conditions that would lead to the change in the product to be controlled, such as the degradation of the product that would make it inadvisable (or dangerous) for consumption. Therefore, the selected bacteria must show a temperature range, growth rate and acid generation capacity such that their growth allows the generation of acid under conditions which correspond to those resulting in product degradation or any other change wanted to be monitored. Hence, the suitable bacteria would be mesophilic bacteria, psychrophilic or psychrotrophic (or even cryophilic for products stored at temperature close to 0°C or at freezing temperature). Mesophilic and psychrotrophic bacteria are preferred for products stored at refrigeration temperature (between 0°C and 5°C) which are very common storage conditions for perishable products, and particularly bacteria which produce at least one acid metabolite between 0°C and 37°C, thus increasing acid production when the temperature increases. In particular, microorganisms which show no-growth or almost no growth at temperature between 1 and 6 Celsius degrees are considered particularly suitable, whereas above 10 Celsius degrees their growth is appreciable by the human eye in few hours when grown in standard laboratory conditions (such as inoculated in culture medium within test tubes or in solid medium culture plates).

The use of different species of in the same indicator is compatible with the invention, which can facilitate adjusting the temperature, growth rate and acid production rate of the bacteria with the time and temperature range in which the product to be controlled shows degradation.

In order to fulfil these conditions, it is preferred the use of lactic acid bacteria are, which are gram positive non-spore cocci or bacillus used in several industrial and agricultural applications, because they are considered safe for human being or even beneficial for health. These specific bacteria produce lactic acid by sugar fermentation leading to a decrease of medium pH, thus facilitating the preservation of perishable foods (dairy and meat). Moreover, these bacteria grow naturally in many perishable foods at a similar temperature and growth rate as microbial flora which degrades the food grows. Hence, these bacteria are suitable to reflect the contamination and the kinetic of the microbial degradation of the products on which the indicator is placed.

It is possible to use lactic acid bacteria such as:
● Streptococcaceae: *Streptococcus lactis, Streptococcus thermophilus, Streptococcus cremoris; Pediococcus, Leuconostoc.*
● Lactobacillaceae: *Lactobacillus casei, Lactobacillus fermentum, Bifidobacterium, Lactobacillus lactis, Lactobacillus acidophilus, Lactobacillus plantarum, Lactobacillus delbrueckii*

For the present invention, experiments were performed preferentially with *Lactobacillus plantarum* and *Lactobacillus delbruecki* due to their greater advantages provided. Indeed, these bacteria present some interesting features: they are non-pathogenic microorganism, they are mesophilic psychrotrophic microorganisms which grow in a range between +5°C and +35°C which is of interest to the invention; they acidify the medium leading to a pH change; they are homofermentator bacteria, aerobic tolerant and therefore they can ferment in the presence or absence of oxygen (which is of interest in encapsulated devices). In addition, they are well known bacteria and commercially available, which minimizes the barriers to market entry.

### - Culture medium

The liquid medium must contain a variety of nutrients required by the microorganism in order to perform the fermentation, besides it must contain a colourimetric indicator which changes its colour when pH falls below a threshold.

The culture medium used contains all elements required for the cell synthesis and metabolites production, taking into account the physic-chemical factors affecting the growth rate, the concentration of nutrients, water activity, pH, oxygen availability and temperature. A summary of the minimum nutrients required by the microorganisms is: carbohydrates (or other similar carbon sources); nitrogen source (e.g. peptone) and preferably inorganic salts and/or vitamins and trace elements. For the correct operation of the device, there must be at least one element from which the bacteria within the device can generate acid that leads to the pH change meaning that the perishable product monitored by the device may not be suitable for consumption.

Lactic acid bacteria can be grown in culture medium containing different carbon sources. Examples of sugars and derivates and other carbohydrates sources include: glycerol, erythritol, D-arabinose, L-arabinose, ribose, D-xylose, L-xylose, adonitol, β-methyl-xilosido, galactose, D-glucose, D-mannose,D-fructose, L-sorbose, rhamnose, dulciton, inositol, mannitol, sorbitol, α-methyl-D-mannoside, α-methyl-D-glucoside, N-acetyl-glucosamine, amygdalin, arbutin, esculin, salicin, cellobiose, maltose, lactose, melibiose, sucrose, trehalose, inulin, melizitosa, D-raffinose, starch, glycogen, xylitol, β-genitibiosa, D-turanose, D-lyxose, D-tatgorsa, D-flucosa, L-fucose, D-arabitol, L-arabitol, gluconate, 2-keto-gluconate, 5-gluconate.

The sugar present within the medium, such as glucose, is needed for the production of lactic acid by the bacteria. Therefore, it is necessary the presence of a sugar such as glucose, fructose, lactose galactose or sucrose , which can be metabolized by the bacteria within the device in order to produce the acidification of the medium.

In the case of the lactic acid bacteria used in the present invention, for instance *Lactobacillus plantarum* and *Lactobacillus delbrueckii,* the sugar present in the culture medium could be the glucose.

A source of nitrogen is also required, for instance tryptone, peptone or any protein origin compound.

Usually when working with lactic acid bacteria, in particular with Lactobacillus genera, a standard medium culture is recommended for the bacteria growth. For instance, ,MRS broth (J.C. de Man, M. Rogosa and M. Elisabeth Sharpe, Appl. Bact. 23, pp. 130-135, 1960). The constituents and typical concentrations of the medium are indicative: peptone (10g/l); meat extract (8g/l), yeast extract (4 g/l), glucose (20g/l) tween80 (1.08ml/l), potassium phosphate (2 g/l), sodium acetate (5g/l), ammonium citrate (2g/l), magnesium sulfate (0.2 g/l); manganese sulfate (0.05 g/l). However, as mentioned in the Examples of present application, this composition of the medium resulted non-adequate to accomplish the acidification of the culture medium wanted due to the buffer effect resulted of some of the components in the MRS medium.

Therefore, a medium lacking of buffering capacity compounds is recommended for the device described in the present invention, in particular when using *Lactobacillus plantarum* and *Lactobaillus delbrueckii.* For this reason the addition of salts either organic or inorganic with buffering capacity is not recommended, such as phosphate salts (PO₄²⁻) or hydrogenphosphate (HPO₄⁻¹), sulfate (SO₄²⁻), carbonate (CO₃²⁻) acetate or citrate among others. This also differs from the patent application US20070275467A1, in which K₂HPO₄ and MgSO₄ are used in the culture medium referring also to the recommended MRS medium.

The composition of the suitable culture medium for an appropriate operation of the device presented in the invention, in particular when using *Lactobacillus plantarum* and *Lactobacillus delbruecki,* is a culture medium comprising:
- Glucose (preferably 30 g/l)
- Peptone (preferably 2 g/l)
- Powdered milk (preferably 10 g/l)
- Yeast extract (preferably 2 g/l)

In a preferred embodiment, salts with buffering capacity are not added to this culture medium.

In addition, the medium must contain an indicator compound which leads to a perceptible change to the human eye depending on the pH, such assubstance that changes its colour depending on pH, that is, a compound which has a different colour for its acidic and basic forms, one of which may be colurless.

### - Indicator of acidification

The device presented in this invention can monitor the disruption of the appropriate storage conditions of the product to which it is adjoined. The break gives rise to the microorganisms growth acceleration, which causes the acidification of the medium that can be visualized by the human eye due to the modification of the pH of the indicator compound which is translated to a change of its color.

It is highly recommended the used of colourimetric indicators in a preferred embodiment, theseindicators are weak acids or bases that show different colours for the basic and acid forms. Thus, the colour of the indicator changes when the pH of the medium is lower or higher that the pKi of the indicator (pH at which the concentration of acid and case forms in the medium is the same).

The acidification indicator used must undergo a clear colour change which is easily visible and perceptible by the human eye. There is a variety of substances that can be used as acidification indicator due to their colour change, by different colours and pKi values, i.e. at different pH value at which the colour changes. The following table shows some indicator compounds examples that can be used for the purpose of the present invention; some characteristics are mentioned, such as the name of the substance, colour in highest pH value (base colour), colour at the lowest pH value ( acid colour) and the range in which the colour change occurs.

**Table 1: pH indicator compounds**

| **Indicator** | **Acid Colour** | **Range** | | **Base Colour** |
|---|---|---|---|---|
| Methyl violet | yellow | 0.0 | 1.6 | blue |
| Cresol red (1st interval) | red | 0.0 | 1.8 | yellow |
| Crystal violet | yellow | 0.0 | 1.8 | blue |
| Malachite green | yellow | 0.2 | 1.8 | blue-green |
| Methyl green | yellow | 0.2 | 1.8 | blue |
| Methanil yellow | red | 1.2 | 2.4 | yellow |
| m-cresol purple (1st interval) | red | 1.2 | 2.8 | yellow |
| metacresol purple | red | 1.2 | 2.8 | yellow |
| Thymol blue (1st interval) | red | 1.2 | 2.8 | yellow |
| 4-o-Tolyl azo-o-toluidine | orange | 1.4 | 2.8 | yellow |
| Orange IV (Tropeoline 00) | red | 1.4 | 3.2 | yellow |
| 2.6-Dinitrophenol | colourless | 1.7 | 4.4 | yellow |
| Benzyl orange | red | 1.9 | 3.3 | yellow |
| 2.4-Dinirophenol | colourless. | 2.4 | 4.0 | yellow |
| Benzopurpurin 4B | violet | 2.2 | 4.2 | red |
| p-Dimethylaminoazobenzene | red | 2.9 | 4.0 | yellow |
| Methyl yellow | Red | 2.9 | 4.0 | yellow |
| Bromochlorophenol blue | yellow | 3.0 | 4.6 | purple |
| Bromophenol blue | mellow | 3.0 | 4.6 | blue violet |
| Tetrabromophenol blue | yellow | 3.0 | 4.6 | blue |
| Congo red | blue | 3.0 | 5.0 | red |
| Methyl orange | red | 3.1 | 4.4 | orange |
| Ethyl orange | red | 3.4 | 4.8 | yellow |
| p-etoxchrysoidine | red | 3.5 | 5.5 | yellow |
| α-naphthyl red | red | 3.7 | 5.0 | yellow |
| bromocresol green | yellow | 3.8 | 5.4 | blue |
| Resazurin | orange | 3.8 | 6.4 | violet |
| 2.5-Dinotrophenol | colourless | 4.0 | 5.8 | yellow |
| Methyl red | red | 4.2 | 6.3 | yellow |
| Azolitmin (litmus) | red | 4.4 | 6.6 | blue |
| Alizarin S red | yellow | 4.6 | 6.0 | red |
| Propyl red | red | 4.6 | 6.6 | yellow |
| Chlorophenol red | yellow | 4.6 | 7.0 | red-purple |
| p-Nitrophenol | colourless | 4.7 | 7.9 | yellow |
| Bromophenol red | yellow | 4.8 | 6.8 | purple |
| Bromocresol purple | yellow | 5.2 | 6.8 | purple |
| P-Nitrophenol | colourless. | 5.4 | 6.6 | yellow |
| Bromothymol blue | yellow | 6.0 | 7.6 | blue |
| Brilliant yellow | yellow | 6.6 | 7.8 | red |
| Phenol red | yellow | 6.6 | 8.4 | red |
| m-Nitrophenol | colourless | 6.6 | 8.6 | yellow |
| Neutral red | red | 6.8 | 8.0 | yellow |
| Rosolic acid | yellow/brown | 6.9 | 8.0 | red |
| Cresol red (2nd interval) | yellow | 7.0 | 8.8 | red |
| α-Naphtolphatlein | brown | 7.3 | 8.7 | green |
| Metacresol purple (2nd interval) | yellow | 7.4 | 9.0 | purple |
| m-Cresol purple (2nd interval) | yellow | 7.4 | 9.2 | purple |
| Orange I (Tropeolin OOO) | yellow | 7.6 | 8.9 | Pink-red |
| thymol blue (2° interval) | yellow | 8.0 | 9.6 | blue |
| thymol red | yellow | 8.0 | 9.6 | Blue |
| Phenolphtalein | colourless | 8.0 | 10.0 | red |
| o-Cresolphtalein | colourless | 8.2 | 9.8 | red |
| Thymolphtalein | colourless | 8.8 | 10.6 | blue |
| Alizarin yellow GG | yellow | 10.0 | 12.0 | orange |
| b-Naphtol violet | yellow | 10.0 | 12.0 | violet |
| Alizarin yellow R | yellow | 10.1 | 12.2 | red |
| Nitramine | colourless | 10.8 | 13.0 | brown |
| Poirrier blue | blue | 11.0 | 13.0 | red |
| Resorcinol yellow (Tropeoline 0) | yellow | 11.1 | 12.7 | orange |
| Benzenesulfonic acid | yellow | 11.4 | 12.6 | orange |
| Indigosulphonic acid | blue | 11.4 | 13.0 | yellow |
| Clayton yellow | yellow | 12.2 | 13.2 | amber |

In the Examples of the present invention chlorophenol red was chosen in order to provide a visual signal the acidification in the medium (initially reddish purple and when the medium is acidified it turns yellow).

Fig.2C shows an example of how the device changes due to the effect of pH change in presence of this colourimetric indicator: the device prior to the acidification of the medium (reddish purple in the original, darker grey in Fig.2C) can be observed on the left side, whereas on the right site the device when the pH has dropped below 4.5 (yellow in the original, lighter grey in Fig.2C) can be seen.

This indicator substance can be added either directly to the microorganism culture medium by subsequently adding the mixture onto the porous matrix, or separately. First option is preferred.

### - Porous matrix

The porous matrix is one of the main aspects of the present invention. The porous matrix is in direct contact with the microorganisms. Therefore, it must be biocompatible and the bacterial concentration must not be affected by the contact. In addition, the matrix must have some specific characteristics related to its structure and porosity that enables to the microorganisms population and the liquid medium to be distributed within, without interfering the fermentation process so it is not altered or slowed. Thus, this TTI device operates as a colourimetric indicator which provides information about possible changes in the cold chain of the product, as shown in Figure 2C. This figure that was mentioned above shows the colour change of the device in case of 6 hours break in the cold chain.

As previously mentioned, these characteristics are achieved by the use of a solid porous matrix which is permeable to water, and whose bases weight is equal or greater than 100 g/m². In addition, its air permeability is preferred to be less than 115 µm/(Pa . s).

The thickness of the porous matrix is limited by the application, due to the TTI device presented in the form of a flat label.

The porous matrix must be made by a biocompatible material that does not prevent the growth of the microorganisms within. These materials could be:
- Vegetable origin fibres such as cotton fibres or cellulose fibres;
- Animal origin materials such as wool, silk or keratin;
- Inorganic matrices such as silicon dioxide or glass fibres;
- Synthetic or semi-synthetic polymeric fibre matrixes, such as polyamide, nylon, poly acrylonitrile, poly vinyl alcohol (PVA), polyesther, polyolephine, polyurethane, ethylene-vynyl acetate copolymer or rayon;
- Porous sponge materials of vegetable, animal, mineral or synthetic origins, such as sponges, which means, those porous materials of synthetic or natural origins, highly capable of retaining liquids.

The matrix can be constituted by one of these natural or artificial materials or by a combination of two or more of them. Moreover, the optimal conditions of this porous matrix relating to basic weight, thickness and permeability can be achieved by stacking porous monolayer sheets, simple sheets, of those materials that can be obtained in this way, until the adequate values are reached.

Moreover, as explained above, the structure of the porous matrix should be such that attenuates the colour presented by the liquid medium so that the colour change of the device from its initial state to its final state is sufficiently obvious so that a person can differentiate with no doubt these two states, which will determine whether a product has been subjected to a breakage in the cold chain conditions or whether it is not the case.

In the trials shown below in the "Examples" section, several studies with different materials and combinations of layers were analysed. It was found that the best results were obtained with a porous matrix preferably prepared from a multilayered cellulose structure formed by 24 g/m² monolayers. In the trials done to verify the most appropriate structure to perceive the colour change of indicating substance, it was observed that the colour difference increased as monolayers were added, until the number reaches about 5-6 layers. From this figure on, the addition of new layers (which may be, for example, 20) does not introduce substantial changes in the colour change of the labels and the differences are kept approximately constant.

If these results about the colour perception are plotted as a function of the matrix weight, it can be observed that the colour differences are increased until the parameter reaches a value of about 100-150 g/m². From these values, the increase in weight does not introduce substantial changes in the colour change of the labels and differences remain fairly constant, although the result seems to be something better in 150 to 250 g/m² interval. This tendency to obtain optimal results or near them, in spite of the grammage is retained even at the higher tested values , close to 500 g/m², such as 480 g/m².

The characteristics and the label functioning will be explained afterwards in detail by means of the examples and figures shown in the following lines.

### EXAMPLES

### - Example 1: Determination of the optimal conditions

### 1.1. Culture medium choice

Since one of the preferred embodiments of the invention device is its use for monitoring food and other perishable products are stored under refrigeration conditions (preferably between 0° and 5°C), lactic acid bacteria were selected as preferred candidates to be used in the invention devices, choosing *Lactobacillus plantarum subsp. plantarum* and *Lactobacillus delbrueckii subsp. delbrueckii* among them. These bacteria were purchased from the Spanish Type Culture Collection, corresponding to strain numbers CECT 748T and CECT 4005 respectively.

In this sense, the trials began by using the standard culture medium for the growth of the mentioned bacteria, this is a type MRS broth, so known for the surname initials of its authors (J. C. de Man, M. Rogosa and M. Elisabeth Sharpe, Appl. Bact. 23, p. 130-135, 1960). The ingredients and typical concentrations of this medium are, in an indicative way, the following: peptone (10 g/l), meat extract 8 g/l), yeast extract 4 g/l), glucose 20 g/l) ; Tween 80 (1.08ml/l), potassium phosphate 2 g/l), sodium acetate (5 g/l), ammonium citrate 2 g/l), magnesium sulfate 0.2g/l); sulfate manganese (0.05 g/l). In this case, the medium was purchased already prepared from Sigma-Aldrich (MRS Broth, product number 69964). The colourimetric indicator Chlorophenol Red (0.1 g/l) was added to this culture medium, prepared by dissolving 0.1 grams of chlorophenol red (purchased from Sigma-Aldrich) in one water liter.

The tests showed that the medium choice is not an obvious process. To this purpose, a single colony was dragged by an inoculating loop from the isolated cultures in plaque. The inoculating loop was introduced into a test tube containing 10 ml of MRS, being the bacterial concentration in the tube on the order of 2 x 10⁸ cfu/ml (cfu: colony forming units). This tube was introduced during 24 hours in an environment with optimal growth conditions, that in the case of selected bacteria, it is a temperature of 37 °C under anaerobiosis. From the preculture incubated for 24 hours, 100 µl were used to inoculate a tube with 50 ml of MRS medium. After 24 hours of incubation a suspension was obtained, which will now be considered as working suspension. After a set of experiments in which the media were inoculated by the *Lactobacillus plantarum or Lactobacillus delbrueckii* working suspension it was found that when subjecting the medium to room temperature (20 °C) in a Stuart Scientific S160D Incubator without stirring, for a period exceeding 12 hours, the expected medium acidification due to the fermentation did not occur and, logically, a colour change was not observed. This situation (a period of time longer than 12 hours at 20 °C) clearly exceeds the conditions of a breakage in the food cold chain, that is why it can be deduced that the standard culture medium prepared to observe the growth of these bacteria is not useful to achieve the goal of the present invention. Therefore, different tests were carried out by reformulating the medium, in order to find the most suitable composition.

After performing various tests, the cause of the MRS medium lack of utility is that there are components in the MRS medium that produce a buffer effect on it, confirming a need of studying the reformulation of the medium.

After several set of experiments in which some ingredients were readjusted, removed and replaced, the culture medium, which best results were obtained with, which serves as an example to illustrate the present invention but that should not be considered limitative, contains carbohydrates as glucose, nitrogen sources like peptone and complex substrates such as yeast extract or powdered milk as well, but preferably does not contain buffer substances which makes the culture medium change of pH difficult.

To carry out the experiments that led to these conclusions on the the culture medium composition, glucose, peptone, yeast extract and, as mentioned before, chlorophenol red compounds were purchased from Sigma-Aldrich. Probiotic Hero Baby Powdered milk was used too.
● Glucose: 30 g/l.
● Peptone: 2 g/l.
● Powdered milk: 10 g/l.
● Yeast extract: 2 g/l.

In addition, the colour indicatin substance has to be considered. In this case, the preferred compund, as mentioned, was:
● Chlorophenol Red: 0,1 g/l.

In these conditions, the colour change was observed in less than 8 hours, although. In fact, the colour change was visible after 6 hours at 20°C.

### 1.2. Choice of the porous matrix

In order to choose the porous matrix, the previous mentioned conditions were taken into account: it has to allow the microorganisms growth and must facilitate the perception of a change in an indicator, preferably a colour based one, due to the acidification of the medium produced by the microorganisms growth, until a point in which the colour change can be observed by the human eye in a clear and obvious way.

The choice of this kind of substrate to be used is not an evident process, since it is necessary a wide variety of experiments until an adequate characteristic porous matrix is achieved.

To find the more appropriate porous matrixes, several time-temperature invention devices were prepared, following the method shown below:

- Preparation of the culture medium: in ultrapure water (18.2 MΩ.cm) the culture medium was prepared together with the indicator in concentrations: glucose: 30 g/l; peptona: 2 g/l; powdered milk: 10 g/l; yeast extract: 2 g/l and chlorophenol red: 0,1 g/l. Glucose, peptone, yeast extract and cholorophenol red were purchased from Sigma-Aldrich Inc., whereas the powdered milk was provided by Hero Inc. Once the mixture was done, it was sterilized by an autoclave at 120°C and so, the culture medium stock solution was obtained.

In addition, a working suspension was prepared after a scaling process, incubating *Lactobacillus plantarum* in sterilized MRS broth medium at 37°C for 24 hours.

Then, 900µl of the culture medium stock solution were extracted in an eppendorf and inoculated with 100 µl of working suspension.

- Preparation of the labels: two polypropylene (Exxon Mobil, ref. 50LL537) plastic sheets were prepared, with rectangular dimensions, 30 x 33 mm, and washed with ethanol.

On the top of one of the sheets, the porous matrix of each experiment was placed. After that, 450 µl of the previously prepared culture medium were deposited, containing approximately 2.3x108 cfu/ml of *Lactobacillus plantarum,* so that the liquid was absorbed until the substrate was saturated. Then, the second plastic sheet was placed over the first one and they were thermally sealed. The obtained labels were used to study the time necessary to observe the colour change depending on the porous matrix.

These labels were kept inactive, this means, at refrigeration temperature (5°C) until they were used.

The different substrates, porous matrix applicants, in which the culture media were deposited to study the effect of the different porosity and basic weight values were, among others: gauze-like tissue (25.5 g/m²), Albet filtering paper (75 g/m²), Versi-Dry ® labsoaker paper (VWR, ref 115-9255) (87.3 g/m²), surface protection paper bench protector VWR® (VWR, ref. 115-9220) (181.8 g/m²), textile tissue (35% cotton, 65% polyesther) (195 g/m²), surface protection filtering paper Fisherbrand® (Fisherbrand, ref W1611C) (211.1 g/m²), compacted cotton fibre discs Demak'Up® (Colhogar) (255 g/m²) multilayered cellulose paper of several grammages (Lorca Medicina Ortopedia S.A. ref. d1 1p60za) (from 24 to 480 g/m²).

In order to study the time necessary to observe the change of colour, a climatic chamber (Compact Climatic Chamber CCLP. INELTEC. S. L.) was used. The labels were subjected to several temperature cycles that simulate the breakage in the cold chain conditions. Concretely, 5°C was considered as a typical refrigeration situation, whereas 20°C was considered as a breakage in the cold chain situation. Therefore, labels where subjected to a 20°C continuous cycle of temperature and the time taken to produce the colour change on them was measured.

It was found that tissues like tissue-type gauze, filter paper Albet, paper labsoaker Versi-Dry®, surface protection Bench Protector VWR®, textile tissue (35% cotton, 65% polyester) or surface protection filtering paper Fisherbrand® did not give optimal results for their use in the invention devices to control of products to be kept under refrigeration when the bacteria used were the *Lactobacillus plantarum* species, since the colour was not distributed homogeneously, or even that colour was imperceptible to the human eye. In other kind of substrates, such as compacted cotton fibre discs, the colour change process was appreciable but it was reached after long time periods that are not adequate for applications requiring a detection of a breakage in the cold chain in a short time, although they can be useful for other applications also compatible with the present invention and use of the devices of itself (for these substrates colour change was produced for periods of time longer than 12 hours at 20°C).

Additionally, it was observed that the multilayered cellulose structure matrix matched with the required characteristics in the present invention, it is biocompatible, the colour is homogeneously distributed and clearly perceptible to human eye and it also permitted the bacteria to acidify the medium in less than 8 hours at 20°C, producing a perceptible change of colour.

Once this multilayered cellulose structure was selected as the preferred medium to be used as the porous matrix, an additional study was carried out to determine its optimal conditions. In this way, the goal was to determine the properties of the tridimensional porous structure, this means, to determine the adequate grammage and porosity that permit to obtain an optimal colour change. A study about the variation of the colour intensity in the labels was carried out, depending on the matrix grammage, what was varied by adding different number of cellulose monolayers, each one of 24 g/m². To this purpose, new labels were prepared as those described in Figs. 2B and 2C, in which the grammage of the porous matrix was varied from 24 to 480 g/m², by adding the number of cellulose monolayers from 1 to 20. Each one of these labels was subjected to different temperature cycles that simulated the breakage in the cold chain conditions (compact climatic chamber CCLP. INELTEC. S. L.), in the same way it was done to identify the most adequate porous material described above. The spectral reflectance of the labels was measured in the UV-VIS range by an spectrophotometer consisted in a integration sphere and using a di:8° geometry. Based on the obtained spectral reflectance curves, CIELAB colour coordinates were determined for the D65 standard illuminant and the 10° standard observer. From these data, the colour differences ΔE*_{ab} (CIE. (2004). Colorimetry 3rd ed (3 ed.). Vienna: Commission Internationale de L'Eclairage) were calculated between the samples presenting the initial colour prior to the colour change and the samples after this change of colour(after the breakage in the cold chain).

The obtained results can be observed in Fig. 3, where the colour differences have been represented both as a function of the number of monolayers of the porous matrix (panel A, where 0 value in the vertical axis corresponds to a label in which there is no porous matrix but it includes the liquid medium with the microorganisms) and as a function of the multilayered porous matrix grammage (panel B).

These results show that the colour difference increases as a function of the number of monolayers, until 5-6 monolayers. From this figure it is observable that the adition of new monolayers does not introduce noticeable changes in the colour change of the labels, so the differences among them keep approximately constant. At the same time, it is also observed that the colour differences are increased as a function of the porous matrix grammage, until a total grammage of 100-150 g/m². From these figures on, the addition of more grammage does not produce noticeable changes in the colour change of the labels and so, the colour changes keep approximately constant.

Therefore, it can be concluded that in the experiments done, the optimal conditions for perceiving visually the colour change are achieved from a porous matrix weighting more than 100 g/m², values being discarded in culture media like those indicated in US2005084948 A1. Under those conditions, the label shows, in a homogeneous way, a clearly perceptible colour to human eye and turns its colour when the breakage in the cold chain is produced.

In order to define other physical properties of the porous matrix, the mentioned cellulose porous matrix with grammage exceeding 100 g/m² was subjected to permeability trials based on the Bendtsen method described in either ISO 5636-4 or UNE 57066-2 standards, obtaining values lower than 115 µm/(Pa . s). It also was subjected to porosity trials following the technique described in either ISO/CD 5636-5 ó UNE 57066-3 standards and the resulting values showed a low average porosity, leaving the results of the trials out of the applications of the corresponding standards and matching with 1.18 seconds.

As a consequence, grammage values (basic weight) and air permeability as well are out of the recommended limits for the biocompatible porous matrixes described in US20050087948A1 document. Despite of this, the device lead both the adequate growth of the microorganisms in itself and the medium acidification to perform a colour change clearly perceptible to human eye, which can be considered as an indicator of the conservation state of the perishable product at which the invention device is adhered. And all of this is done without the need of using any texturising component or additive to immobilizate the microorganisms and / or the nutrients, this means, as explained previously, all those components that give certain viscosity to a liquid substrate and that can own properties related to water retention, as those used in the described culture media for time-temperature devices similar to the present invention as, for example, the described in patent document US20070275467A1.

### - Example 2: illustrative example of the labels functioning

After the media and porous matrix optimizing studies presented previously, in the following lines an illustrative example of the functioning of one of the present invention devices is described. This example must not be considerated as a limitation in the scope of this invention.

Several labels were prepared in parallel, as has been described in Example 1, point 1.2., by preparing the same culture medium with microorganisms and indicating medium and using, at the same time, polypropylene plastic sheets, previously washed in ethanol, to cover the porous matrix and to isolate it from the outside.

As porous matrix, a cellulose porous matrix weighting 480 g/m² was used, with dimensions 20 x 23 mm, which was placed over one of the plastic sheets. Then, 450µl of the inoculated medium were deposited over the mentioned substrate, in a way that the liquid was absorbed and that the porous substrate was saturated. The second plastic sheet was placed over the substrate and they were thermally sealed.

The labels kept inactive at 5°C and they were subjected to a continuous temperature cycle at 20°C afterwards. The process of colour change was considered finished after 8 hours, when the labels fully change their colour. Fig. 4 shows some pictures corresponding to one of the porous matrix of a label before beginning the incubation at 20°C (a) and after the colour change (b). The colour change is noticeable and simply observable, as part (a) (dark gray in Fig. 4) is reddish-purple in the original whereas part (b) is shown as ambar-yellow in the original, which can be easily distinguishable from the previous state.

With the objective of checking whether these labels worked as proper TTI devices and that they enjoyed a memory effect capable of registering in a cumulative way the temperature excess, the labels were subjected to a discontinuous and variable cycle (cycle 2) between the critical temperature of 20°C and the refrigeration temperature (5°C). This was 3 hours at 20°C, followed by 3 hours at 5°C and then, 3 hours at 20°C, pictures were taken at 3, 6 and 9 hours. The obtained pictures are shown as part of Fig. 5 (pictures in lower row, "Cycle 2"); on top of these pictures of the "Cycle 2", as a comparison, the pictures obtained from a label subjected to a continuous cycle at 20°C have been placed (pictures in higher row, "Cycle 1"). As can be observed in Fig. 5, the memory effect is clear, as a delay in the turn process is appreciated corresponding to the time being subjected to refrigeration temperatures. These results confirm that these devices can work as TTI labels to monitor breakages in the cold chain of products that have to be conserved at refrigeration temperatures, and that their use as TTI labels is compatible with the present invention and falls into the scope of the invention.

## Claims

1. A device for monitoring the time and temperature history of a product, **characterised in that** it comprises:
a) a first sheet of plastic material that is impermeable to water;
b) a solid porous matrix which is permeable to water whose basis weight is equal or greater than 100 g/m², which is placed on the surface of the first sheet of plastic material;
c) a second sheet of plastic material, which is impermeable to water and transparent or translucent over at least a part of its surface, located over the solid porous matrix, in such a way that the solid porous matrix is covered by the second sheet of plastic material and is visible through the transparent or translucent section of the said second sheet of plastic material.

2. The device according to claim 1, wherein the first sheet of plastic material contains an adhesive film over the surface of the sheet which is not in contact with the porous matrix.

3. The device according to claim 1 or 2, wherein the solid porous matrix is a multilayered cellulose structure with a basis weight equal to or greater than 100 g/m².

4. The device according to claim 3, wherein the basis weight of the multilayered cellulose structure ranges from 100 to 480 g/m², or is equal to 480 g/m².

5. The device according to claim 3, which comprises at least 5 cellulose layers of 24 g/m² each.

6. The device according to any one of the claims 1 to 5, wherein the solid porous matrix has a permeability to the air of less than 115 µm (Pa.s).

7. The device according to claim 6, wherein the solid porous matrix has a permeability to the air greater than 17.40 µm (Pa.s).

8. The device according to any one of the claims 1 to 5, wherein the solid porous matrix has an average porosity greater than 1.18 seconds.

9. The device according to any of the claims 1 to 8, wherein the two sheets of plastic material are sealed together around the whole perimeter of the device, and which also includes:
d) microorganisms distributed within the porous matrix;
e) a culture medium for said microorganisms;
f) an indicator compound which undergoes a change that is perceptible to the human eye depending on a change in the medium caused by the growth of the microorganisms.

10. The device according to claim 9, wherein
i) the microorganisms produce an acid or metabolite which increases the acidity of the medium during their growth, development or metabolic activity, and
ii) the indicator compound is a colour indicator which has different colours for its acidic and basic forms, one of which may be colourless.

11. The device according to claim 9 or 10, wherein the microorganisms are lactic bacteria selected from the group of *Streptococcus lactis, Streptococcus thermophilus, Streptococcus cremoris, Pediococcus spp., Leuconostoc spp., Lactobacillus casei, Lactobacillus fermentum, Bifidobacterium spp., Lactobacillus lactis, Lactobacillus acidophilus, Lactobacillus plantarum* and *Lactobacillus delbrueckii,* or combinations thereof.

12. The device according to claim 11, wherein the culture medium comprises glucose, peptone, powdered milk and yeast extract, and lacks substances with buffering capacity.

13. The device according to claim 12, wherein:
i) the composition of the culture medium is:
- glucose: 30 g/l;
- peptone: 2 g/l
- powdered milk: 10 g/l
- yeast extract: 2 g/l,
ii) the indicator compound is a colour indicator which has different colours for its acidic and basic forms, one of which may be colourless, and
iii) the microorganisms are lactic bacteria which belong to the species *Lactobacillus plantarum* or *Lactobacillus delbrueckii.*

14. The device according to any one of the claims 10 to 13, wherein the colour indicator is chlorophenol red.

15. The device according to claim 14, in which:
a) the first sheet of plastic material that is impermeable to water and which contains a film of adhesive over one of its surfaces, is polypropylene;
b) the solid porous matrix which is permeable to water is a multilayered cellulose structure with a basic weight of 480 g/m²;
c) the second sheet of plastic material that is impermeable to water and which is transparent over at least a part of its surface, is polypropylene;
d) the microorganisms distributed within the porous matrix belong to the species *Lactobacillus plantarum;*
e) the culture medium of the microorganisms includes glucose (30 g/l), peptone (2 g/l), powdered milk (10 g/l), yeast extract (2 g/l), and
f) that it also includes chlorophenol red (0.1 g/l), as an indicator compound.

16. Use of a device according to any one of the claims 9 to 15 for controlling the time-temperature history of a product to which the device is adjoined and which must be kept below a certain temperature.

17. Use according to claim 16, wherein the change of the indicator compound that is perceptible to the human eye indicates that:
a) a certain change in the conditions of the product has occurred,
b) the product has been kept above a threshold temperature for a time longer than a preset value,
c) the product is no longer in a suitable condition for consumption,
d) the product must be consumed immediately, or
e) that the conditions of conservation intended for the product have not been complied with.

18. Use according to claim 16 or 17, wherein the indicator compound is an indicator of pH with different colours of its acidic and basic forms, one of which may be colourless.

19. Use according to claim 17 or 18, wherein breaks in the cold chain of a product are monitored, which product has to be kept under refrigeration conditions.

20. Use according to any one of the claims 16 to 19, wherein the indicator device is a device as claimed in claim 15.
